# EUROPÄISCHE PATENTSCHRIFT

(11) **EP 0 483 052 B1**
(45) Veröffentlichungstag und Bekanntmachung des Hinweises auf die Patenterteilung: **12.03.1997**
(21) Anmeldenummer: 91810726.9
(22) Anmeldetag: 11.09.1991
(51) Int. Cl.: C07D 401/06, A01N 43/54, A01N 43/50

(54) **Picolinoxide**
Picolinoxides
Oxydés de picoline

(30) Priorität: 18.09.1990 CH 3016/90
(43) Veröffentlichungstag der Anmeldung: 29.04.1992
(73) Patentinhaber: CIBA-GEIGY AG, 4002 Basel (CH)
(72) Erfinder: Kristiansen, Odd, Dr., CH-4313 Möhlin (CH); Gsell, Laurenz, Dr., CH-4056 Basel (CH); Maienfisch, Peter, Dr., CH-4118 Rodersdorf (CH)

(56) Entgegenhaltungen:
- EP-A- 0 192 060
- EP-A- 0 259 738
- EP-A- 0 292 822
- EP-A- 0 296 453
- EP-A- 0 315 826
- EP-A- 0 316 845
- EP-A- 0 386 565

## Beschreibung

Die vorliegende Erfindung betrifft Derivate von 3-Picolin-N-oxid, Verfahren zu ihrer Herstellung, Schädlingsbekämpfungsmittel, welche diese Verbindungen enthalten, Verfahren zur Bekämpfung von Schädlingen sowie die Anwendung dieser Verbindungen bei der Kontrolle von Schädlingen, vorzugsweise von Insekten.

Die erfindungsgemässen 3-Picolin-N-oxid-Derivate entsprechen der Formel I 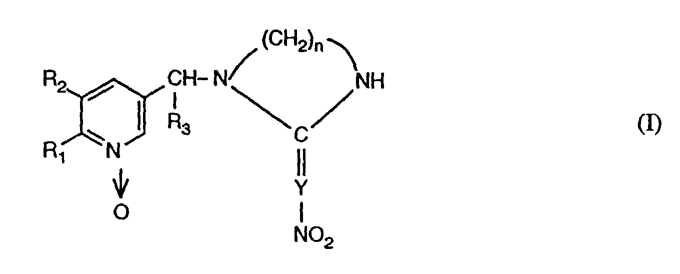 worin
R₁ und R₂ unabhängig voneinander Wasserstoff oder Halogen,
R₃ Wasserstoff oder C₁-C₄-Alkyl,
n die Zahl zwei oder drei und
Y Stickstoff oder die Methingruppe bedeuten.

Aus der Literatur sind heterocyclische Verbindungen, welche eine Nitroguanidinstruktur oder Nitroäthylenstrukturen enthalten, aus der EP-A-192 060 als Insektizide bekannt. Die biologischen Eigenschaften dieser Verbindungen vermögen jedoch bei der Schädlingsbekämpfung nicht voll zu befriedigen.

Die Verbindungen der Formel I können in der zur Formel I tautomeren Formel Ia auftreten.

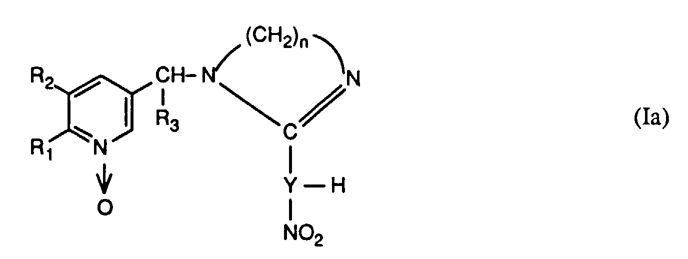

Die erfindungsgemässe Formel I ist hierin so zu verstehen, dass die Formel Ia in der Schreibweise der Formel I eingeschlossen ist.

Ferner umfasst die Schreibweise der erfindungsgemässen Formel I auch die möglichen E- und Z-Isomeren der Formel Ib und Ic

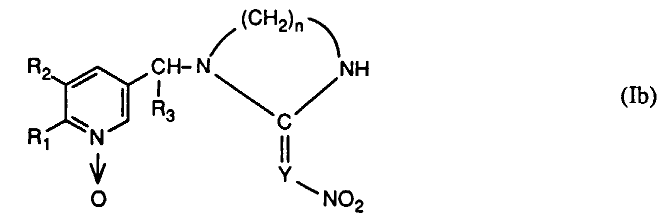

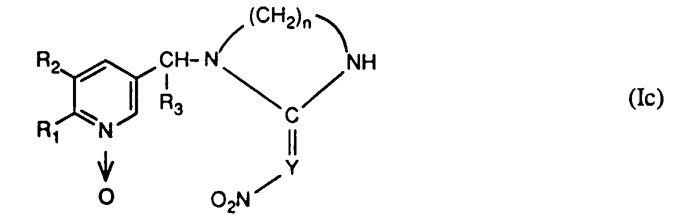

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor, Chlor und Brom, insbesondere aber Chlor bevorzugt sind.

Die als Substituenten in Betracht kommenden Alkylreste können geradkettig oder verzweigt sein. Als Beispiele solcher Alkyle seien Methyl, Aethyl, Propyl, Isopropyl, Butyl, i-Butyl, sek.-Butyl oder tert.-Butyl genannt.

Unter den Verbindungen der Formel I sind solche Untergruppen herauszuheben, in denen entweder
a) R₁ und R₂ unabhängig voneinander für Wasserstoff oder Chlor stehen, oder
b) R₃ Wasserstoff bedeutet.

Aus der Untergruppe a) sind solche Verbindungen bevorzugt, worin R₁ Chlor und R₂ Wasserstoff bedeuten.

Eine besonders bevorzugte Gruppe von Verbindungen der Formel I ist dadurch gekennzeichnet, dass R₁ Chlor und R₂ und R₃ Wasserstoff bedeuten.

Als bevorzugte Einzelverbindungen der Formel I sind zu nennen:
3-(2-Nitromethyliden-imidazolidin-1-ylmethyl)-pyridin-N-oxid,
2-Chlor-5-(2-nitromethyliden-imidazolidin-1-ylmethyl)-pyridin-N-oxid und
2-Chlor-5-(2-nitroimino-imidazolidin-1-ylmethyl)-pyridin-N-oxid.

Die erfindungsgemässen Verbindungen der Formel I können in Analogie zu bekannten Verfahren hergestellt werden. Beispielsweise erhält man die Verbindung der Formel I, indem man ein Halogen-3-picolin-N-oxid der Formel II 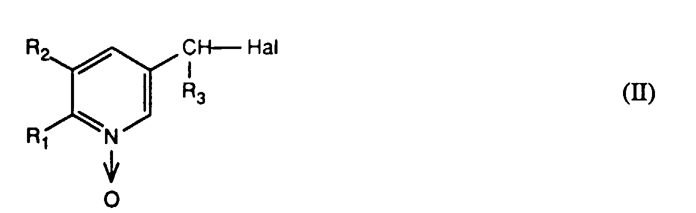 worin R₁, R₂ und R₃ die unter Formel I gegebenen Bedeutungen haben und Hal für Halogen, vorzugsweise Chlor oder Brom steht, in Gegenwart eines säurebindenden Mittels mit einem cyclischen Amin der Formel III 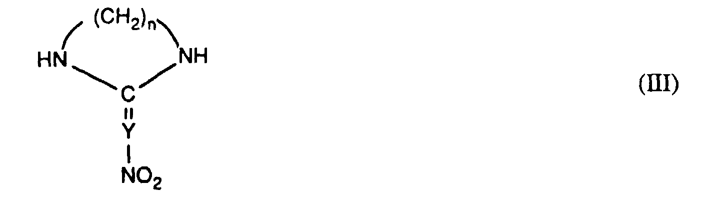 worin n und Y die unter Formel I gegebenen Bedeutungen haben, umsetzt.

Die Durchführung des erfindungsgemässen Verfahrens (II + III → I) erfolgt mit Vorteil in einem inerten Lösungsmittel bei Temperaturen zwischen -10°C und + 100°C, insbesondere zwischen 0°C und +80°C. Als Lösungsmittel eignen sich in besonderer Weise polare aprotische Lösungsmittel wie Dimethylacetamid, 1-Methylpyrrolidon, Dimethylsulfoxid, Sulfolan, Tetrahydrofuran, Dioxan, Acetonitril oder Dimethylformamid. Als säurebindende Mittel eignen sich Carbonate wie Natriumcarbonat, Kaliumcarbonat, Hydroxide wie Natriumhydroxid oder Kaliumhydroxid oder Hydrogencarbonate wie Natriumhydrogencarbonat oder Kaliumhydrogencarbonat. Weiter eignen sich als säurebindende Mittel tertiäre organische Amine wie Triäthylamin oder Diäthylanilin. Auch Hydride wie Natriumhydrid können als säurebindende Mittel eingesetzt werden. Beispielsweise kann beim Einsatz von Natriumhydrid in Acetonitril oder Dimethylformamid die Reaktionstemperatur auf 0°C bis +25°C herabgesetzt werden, ohne dass sich dadurch die Reaktionszeit wesentlich verlängert. Mit Vorteil kann die Umsetzung auch durch den Zusatz eines katalytisch wirkenden Salzes wie Cäsiumchlorid erleichtert werden.

Die Zwischenprodukte der Formeln II und III, sind bekannt oder können in Analogie zu bekannten Verfahren hergestellt werden.

Beispielsweise erhält man die Halogen-3-picolin-N-oxide der Formel II, indem man die Halogen-3-picolin der Formel IV 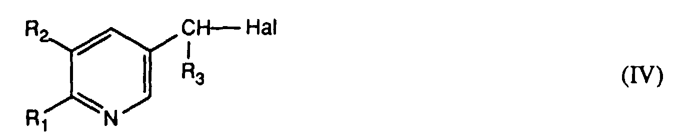 mit einem Oxidationsmittel, wie einer organischen Persäure, z.B. 3-Chlorperbenzoesäure, oder Wasserstoffperoxid, oder einer in situ erzeugten Persäure, wie in den Systemen H₂O₂/Eisessig oder H₂O₂/F₃C-COOH, umsetzt.

Die cyclischen Amine der Formel III werden zum Beispiel erhalten, indem man Nitroguanidin der Formel V 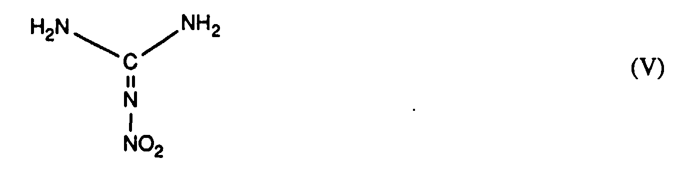 mit einem bifunktionellen Amin der Formel

H₂N-(CH₂)ₙ-NH₂

umsetzt, oder indem man das Nitro-bismercaptoäthylen der Formel VI 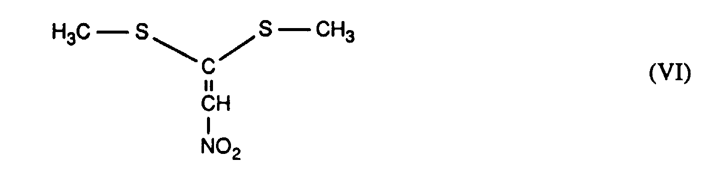 mit dem bifunktionellen Amin der obigen Formel umsetzt.

Es wurde nun gefunden, dass die erfindungsgemässen Verbindungen der Formel I bei günstiger Warmblüter-, Fisch-, Vogel- und Pflanzenverträglichkeit wertvolle Wirkstoffe in der Schädlingsbekämpfung sind. Insbesondere betrifft die Anwendung der erfindungsgemässen Wirkstoffe Insekten, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren Schaden verursachen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung der Orthoptera zum Beispiel
Blatta spp., Blattella spp., Gryllotalpa spp., Leucophaea maderae, Locusta spp.,
Periplaneta spp. und Schistocerca spp.;
aus der Ordnung der Isoptera zum Beispiel
Reticulitermes spp.;
aus der Ordnung der Psocoptera zum Beispiel
Liposcelis spp.;
aus der Ordnung der Anoplura zum Beispiel
Haematopinus spp., Linognathus spp. Pediculus spp., Pemphigus spp. und Phylloxera spp.;
aus der Ordnung der Mallophaga zum Beispiel
Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp. Eurygaster spp.
Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera zum Beispiel
Ceratophyllus spp., Xenopsylla cheopis; und
aus der Ordnung der Thysanura zum Beispiel
Lepisma saccharina.

Als besonderer Vorteil der erfindungsgemässen Wirkstoffe der Formel I hat sich herausgestellt, dass bei der Behandlung der Kulturpflanzen die an ihnen lebenden Nützlinge weitgehend geschont werden. Diese Nützlinge sind in der Regel Insekten und Spinnentiere der Ordnung Acarina, welche bei der Kultivierung von Kulturpflanzen nützlich oder sogar notwendig sind, indem sie Kulturpflanzenschädlinge vertilgen oder beispielsweise für die Befruchtung der Kulturpflanzen unentbehrlich sind. In diesem Zusammenhang sollen insbesondere die Raubmilben der Familie Cheyletidae und der dazugehörenden Gattung Amblyseius wie die Spezies Amblyseius fallacis; die Schlupfwespen der Familie Ichneumonidae; die Marienkäfer der Familie Coccinellidae; Spinnen der Familie Lycosidae, und der dazugehörenden Gattung Lycosa; Spinnen der Familie Oxypidae und der dazugehörenden Gattung Oxyopes; die Bienen, insbesondere die Honig-sammelnden Bienen und schliesslich im Sinne der Naturhygiene auch die Ameisen erwähnt werden.

Ein weiterer Vorteil der erfindungsgemässen Verbindungen der Formel I liegt in ihrem vorteilhaften Umweltverhalten. Es hat sich gezeigt, dass unerwünschte Rückstände von Verbindungen der Formel I im Boden leicht abgebaut werden. Insbesondere zeigt sich, dass die Abbauzeiten der erfindungsgemässen Wirkstoffe deutlich kürzer sind, als entsprechende Werte für die aus dem Stand der Technik bekannten Nitroguanidine und Nitroäthylene der EP-A-192060.

Vorzugsweise eignen sich die Wirkstoffe der Formel I zur Kontrolle von Schädlingen in Gemüse-, Reis- und Zuckerrübenkulturen, wie Blattläusen und Reiszikaden.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlen-wassertoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen der Formel I auch für den Einsatz bei der Behandlung von Saatgut. Dabei kann sowohl das Saatgut vor dem Säen mit dem Wirkstoff oder einer den Wirkstoff enthaltenden Formulierung behandelt oder gebeizt werden, als auch der Wirkstoff beim Säen in die Saatfurche appliziert werden.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen C₈ bis C₁₂ von Alkylbenzolen wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, und Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetanolalkohol, starke polare Lösungsmittel, wie N-Methyl-2-pyrrolidon, Dimethylsulfoxid oder Dimethylformamid oder Wasser, Pflanzenöle, wie Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren (C₁₀-C₂₂), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:
"Mc Cutcheon's Detergents and Emulsifiers Annual", Mc Publishing Corp., Glen Rock, NJ, USA, 1988",
H. Stache, "Tensid-Taschenbuch", 2. Aufl., C. Hanser Verlag München, Wien 1981.
M. and J. Ash. "Encyclopedia of Surfactants", Vol. I-III, Chemical Publishing Co., New York, 1980- 1981.

Die pestiziden Zubereitungen enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 25 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen: (% = Gewichtsprozent)

| Emulgierbare Konzentrate: | |
|---|---|
| Aktiver Wirkstoff: | 1 bis 90 %, bevorzugt 5 bis 20 % |
| oberflächenaktives Mittel: | 1 bis 30 %, vorzugsweise 10 bis 20 % |
| flüssiges Trägermittel: | 5 bis 94 %, vorzugsweise 70 bis 85 % |

| Stäube: | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

| Suspension-Konzentrate: | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

| Benetzbare Pulver: | |
|---|---|
| Aktiver Wirkstoff: | 0,5 bis 90 %, vorzugweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

| Granulate: | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, z.B. gegebenenfalls epoxidierte Pflanzenöle (epoxidiertes Kokosnussöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung.

### Herstellungsbeispiele

### Beispiel H1: 2-Chlor-5-(2-nitroimino-imidazolidin-1-ylmethyl)-pyridin-N-oxid

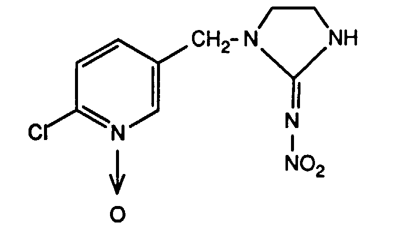

0,85 g einer 55 %igen Natriumhydrid-Dispersion in Mineralöl werden portionsweise einer Lösung von 2,7 g 2-Nitroimino-imidazolidin in 70 ml Acetonitril zugefügt. Das Gemisch wird für 1,5 Stunden unter einer Stickstoffatmosphäre gerührt und anschliessend portionsweise mit 3,72 g 2-Chlor-5-chlormethyl-pyridin-N-oxid versetzt. Das Reaktionsgemisch wird für 16 Stunden bei Raumtemperatur gerührt und anschliessend filtriert. Durch Abkühlen des Filtrats auf 0°C wird das Rohprodukt auskristallisiert. Dieses Produkt wird abgetrennt und mit Diäthyläther gewaschen. Man erhält so reines 2-Chlor-5-(2-nitroimino-imidazolidin-1-ylmethyl)-pyridin-N-oxid, Smp. 200°C (unter Zersetzung).

In analoger Weise können die in der folgenden Tabelle aufgelisteten Verbindungen der Formel I hergestellt werden.

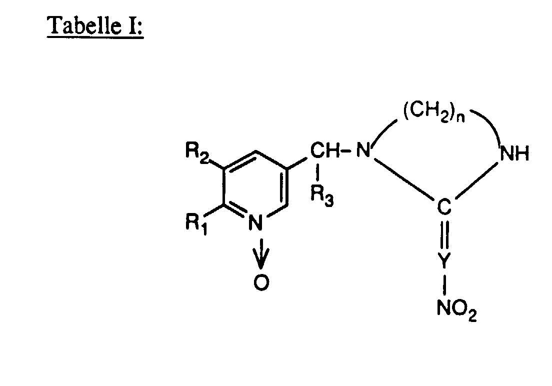

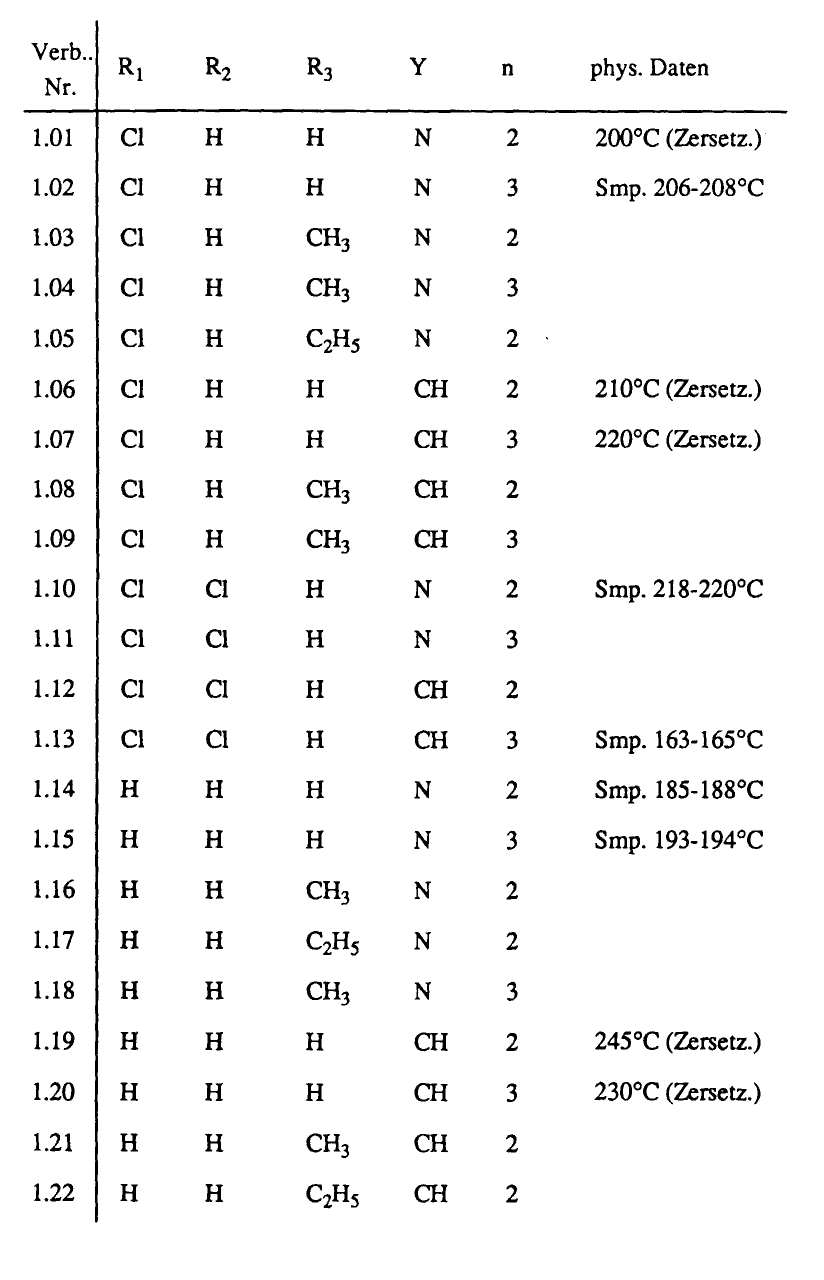

### Formulierungsbeispiele (% = Gewichtsprozent)

| Beispiel F1: Emulsions-Konzentrate | a) | b) | c) |
|---|---|---|---|
| Wirkstoff aus Tabelle 1 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff aus Tabelle 1 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirstoff aus Tabelle 1 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschliessend im Vakuum abgedampft.

| Beispiele F4: Stäubemittel | a) | b) |
|---|---|---|
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff Nr.1.01 oder 1.06 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | |
|---|---|
| Wirkstoff Nr. 1.01 oder 1.06 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 1.19 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Beispiel F8: Extruder-Granulat | |
|---|---|
| Wirkstoff Nr. 1.19 | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| Beispiel F9: Umhüllungs-Granulat | |
|---|---|
| Wirkstoff Nr. 1.06 | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| Beispiel F10: Suspensions-Konzentrat | |
|---|---|
| Wirkstoff Nr. 1.01 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| Silikonöl in Form einer 75 %igen wässrigen Emulsion | 1 % |
| Wasser | 32 % |

Der fein gemahlene Wirkstoff oder die Wirkstoffkombination wird mit den Zusatzstoffen innig vemischt. Man erhält so ein Suspensions-Konzentrat, aus welchem durch Verdünnen mit Wasser Suspensionen jeder gewünschten Konzentration hergestellt werden können.

### Biologische Beispiele

### Beispiel B1: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Nilaparvata lugens in diesem Test. Insbesondere die Verbindungen 1.01, 1.06, 1.07, 1.14, 1.15, 1.19 und 1.20 zeigen eine Wirkung über 80 %.

### Beispiel B2: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Nephotettix cincticeps in diesem Test. Insbesondere die Verbindungen 1.01, 1.06, 1.07, 1.15, 1.19 und 1.20 zeigen eine Wirkung über 80 %.

### Beispiel B3: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Diabrotica balteata in diesem Test. Insbesondere die Verbindungen 1.01, 1.02, 1.06 und 1.20 zeigen eine Wirkung über 80 %.

### Beispiel B4: Wirkung gegen Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Verbindungen der Tabelle 1 zeigen eine gute Wirkung gegen Heliothis virescens in diesem Test. Insbesondere die Verbindungen 1.01 und 1.06 zeigen eine Wirkung über 80 %.

### Beispiel B5: Wirkung gegen Spodoptera littoralis Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des dritten Stadiums von Spodoptera littoralis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Verbindungen der Tabelle 1 zeigen eine gute Wirkung gegen Spodoptera littoralis in diesem Test. Insbesondere die Verbindungen 1.06 und 1.19 zeigen eine Wirkung über 80 %.

### Beispiel B6: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Aphis craccivora in diesem Test. Insbesondere die Verbindungen 1.01, 1.02, 1.06, 1.07, 1.15, 1.19 und 1.20 zeigen eine Wirkung über 80 %.

### Beispiel B7: Systemische Wirkung gegen Nilaparvata lugens

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Nilaparvata lugens in diesem Test. Insbesondere die Verbindungen 1.01, 1.02, 1.06, 1.07, 1.10, 1.14, 1.15, 1.19 und 1.20 zeigen eine Wirkung über 80 %.

### Beispiel B8: Systemische Wirkung gegen Nephotettix cincticeps

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Nephotettix cincticeps in diesem Test. Insbesondere die Verbindungen 1.01, 1.06, 1.07, 1.15, 1.19 und 1.20 zeigen eine Wirkung über 80 %.

### Beispiel B9: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Myzus persicae in diesem Test. Insbesondere die Verbindungen 1.01, 1.06, 1.07, 1.19 und 1.20 zeigen eine Wirkung über 80 %.

### Beispiel B10: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten von Anthonomus grnadis besiedelt und in einen Plastikbehälter gegeben. 3 Tage später erfolgt die Auswertung. Aus dem Vergleich der Anzahl toter Käfer und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Verbindungen der Tabelle 1 zeigen eine gute Wirkung gegen Anthonomus grandis in diesem Test. Insbesondere die Verbindung 1.06 zeigt eine Wirkung über 80 %.

### Beispiel B11: Wirkung gegen Bemisia tabaci

Buschbohnen-Pflanzen werden in Gazekäfige gestellt und mit Adulten Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.

Verbindungen gemäss Tabelle 1 zeigen in diesem Test gute Wirkung gegen Bemisia tabaci. Insbesondere die Verbindung 1.06 zeigt eine Wirkung über 80 %.

### Beispiel B12: Wirkung gegen Myzus persicae

Erbsenkeimlinge werden mit Myzus persicae infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Verbindungen der Tabelle 1 zeigen eine gute Wirkung gegen Myzus persicae in diesem Test. Insbesondere die Verbindungen 1.01, 1.06 und 1.19 zeigen eine Wirkung über 80 %.

### Beispiel B13: Wirkung gegen Amblyseius fallacis (Raubmilbe)

Junge Bohnenpflanzen werden mit einer wässrigen Emulsionsspritzbrühe, die 50 ppm des zu prüfenden Wirkstoffes enthält, besprüht. 0, 2 und 4 Tage nach der Behandlung werden 40 Weibchen von Amblyseius fallacis auf die Blätter gesetzt und mit Spinnmilben der Art Tetranychus urticae gefüttert. 4 Tage nach dem Ansatz der Raubmilben erfolgt die Auswertung. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.

Die Verbindungen der Tabelle 1 zeigen in diesen Test eine Wirkung von höchstens 20 %. Insbesondere die Verbindung 1.01 zeigt auch bei einer Konzentration von 400 ppm in allen drei Versuchsreihen keine Mortolitätseffekte von über 20 % auf Amblyseius fallacis, während die aus dem Stand der Technik bekannte Verbindung 2-Chlor-5-(2-nitroimino-imiazolidin-1-ylmethyl)pyridin jeweils bereits bei 50 ppm eine Schädigung der Raubmilbenpopulation von mehr als 50 % verursacht.

### Beispiel B14: Wirkung gegen Schmeissfliegen Lucilia cuprina

Frisch abgelegte Eier der Schmeissfliegenart Lucilia cuprina werden in kleinen Portionen (30-50 Eier) in Reagenzgläser gegeben, in denen zuvor 4 ml Nährmedium mit 1 ml Testlösung, die 16 ppm des zu prüfenden Wirkstoffes enthält, vermischt worden sind. Nach Beimpfung des Kulturmediums werden die Testgläser mit einem Wattestopfen verschlossen und im Brutschrank bei 30°C 4 Tage lang bebrütet. Im unbehandelten Medium entwickeln sich bis zu diesem Zeitpunkt ca. 1 cm lange Larven (Stadium 3). Ist die Substanz aktiv, so sind die Larven zu diesem Zeitpunkt entweder tot oder deutlich zurückgeblieben. Die Auswertung erfolgt nach 96 Stunden.

Verbindungen der Tabelle 1 zeigen eine gute Wirkung gegen Lucilia cuprina in diesem Test. Insbesondere die Verbindungen 1.01, 1.06 und 1.07 zeigen eine Wirkung über 80 %.

### Beispiel B15: Wirkung gegen Ctenocephalides felis

20 bis 25 Floheier werden in eine waagrecht stehende 50 ml-Zellkulturflasche gegeben, in der zuvor 15 g Flohlarven-Nährmedium, welches 100 ppm des zu prüfenden Wirkstoffs enthält, vorgelegt wurde. Die Testflaschen werden in einem Brutschrank bei 26-27°C und 60-70 % Luftfeuchtigkeit bebrütet. Nach 21 Tagen wird die Anwesenheit von adulten Flöhen, nicht geschlüpften Puppen und Larven überprüft.

Verbindungen der Tabelle 1 zeigen eine gute Wirkung gegen Ctenocephalides felis in diesem Test.

## Patentansprüche (Patentansprüche für folgende(n) Vertragsstaat(en): BE, CH, DE, DK, FR, GB, GR, IT, LI, LU, NL)

1. Ein 3-Picolin-N-oxid der Formel 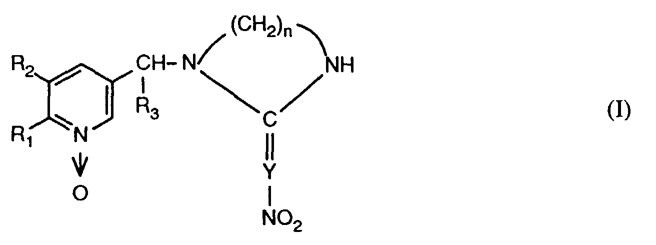 worin
R₁ und R₂ unabhängig voneinander Wasserstoff oder Halogen,
R₃ Wasserstoff oder C₁-C₄-Alkyl,
n die Zahl zwei oder drei und
Y Stickstoff oder die Methingruppe bedeuten.

2. Eine Verbindung gemäss Anspruch 1 der Formel I, worin R₁ und R₂ unabhängig voneinander Wasserstoff oder Chlor bedeuten.

3. Eine Verbindung gemäss Anspruch 2 der Formel I, worin R₁ Chlor und R₂ Wasserstoff oder Chlor bedeutet.

4. Eine Verbindung gemäss Anspruch 1 der Formel I, worin R₃ Wasserstoff bedeutet.

5. Eine Verbindung gemäss Anspruch 1 der Formel I, worin R₁ Chlor und R₂ und R₃ Wasserstoff bedeuten.

6. Eine Verbindung gemäss Anspruch 1, ausgewählt aus der Reihe:
3-(2-Nitromethyliden-imidazolidin-1-ylmethyl)-pyridin-N-oxid,
2-Chlor-5-(2-nitromethyliden-imidazolidin-1-ylmethyl)-pyridin-N-oxid und
2-Chlor-5-(2-nitroimino-imidazolidin-1-ylmethyl)-pyridin-N-oxid.

7. Verfahren zur Herstellung einer Verbindung der Formel I gemäss Anspruch 1, dadurch gekennzeichnet, dass man ein Halogen-3-picolin-N-oxid der Formel 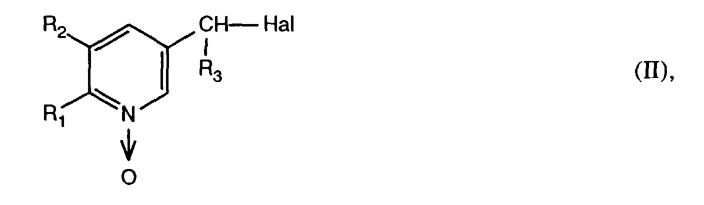 worin R₁, R₂ und R₃ die unter Formel I gegebenen Bedeutungen haben und Hal für Halogen steht, in Gegenwart eines säurebindenden Mittels mit einem cyclischen Amin der Formel 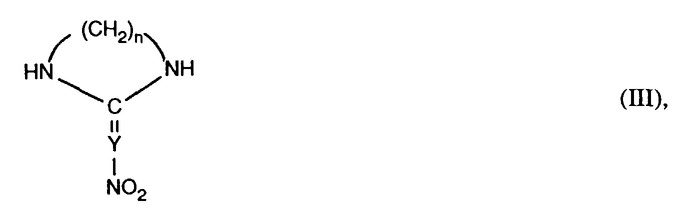 worin n und Y die unter Formel I gegebenen Bedeutungen haben, umsetzt.

8. Schädlingsbekämpfungsmittel, welches als aktive Komponente mindestens ein 3-Picolin-N-oxid der Formel I gemäss Anspruch 1 enthält.

9. Mittel gemäss Anspruch 8, dadurch gekennzeichnet, dass es neben dem Wirkstoff der Formel I noch mindestens einen Trägerstoff enthält.

10. Verfahren zur Bekämpfung von pflanzenschädigenden Insekten, dadurch gekennzeichnet, dass man die Schädlinge oder ihren Lebensraum mit einer wirksamen Menge einer Verbindung gemäss Anspruch 1 der Formel I behandelt.

11. Eine Verbindung gemäss Anspruch 1 der Formel I zur Anwendung in einem Verfahren zur Bekämpfung von Schädlingen an Tieren und Pflanzen.

12. Verbindung zur Anwendung gemäss Anspruch 11, dadurch gekennzeichnet, dass es sich bei den Schädlingen um pflanzenschädigende Insekten handelt.

## Patentansprüche (Patentansprüche für folgende(n) Vertragsstaat(en): ES)

1. Verfahren zur Herstellung eines 3-Picolin-N-oxids der Formel 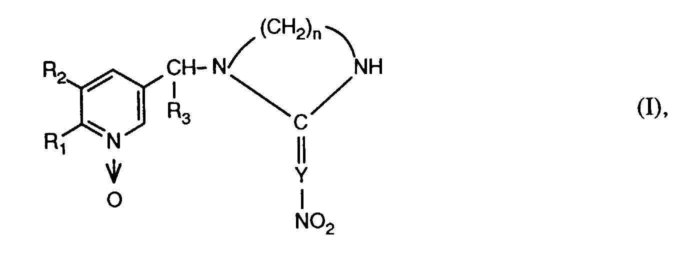 worin
R₁ und R₂ unabhängig voneinander Wasserstoff oder Halogen,
R₃ Wasserstoff oder C₁-C₄-Alkyl,
n die Zahl zwei oder drei und
Y Stickstoff oder die Methingruppe bedeuten, dadurch gekennzeichnet, dass man ein Halogen-3-picolin-N-oxid der Formel 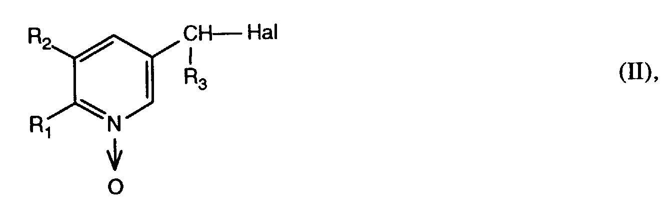
worin R₁, R₂ und R₃ die unter Formel I gegebenen Bedeutungen haben und Hal für Halogen steht, in Gegenwart eines säurebindenden Mittels mit einem cyclischen Amin der Formel 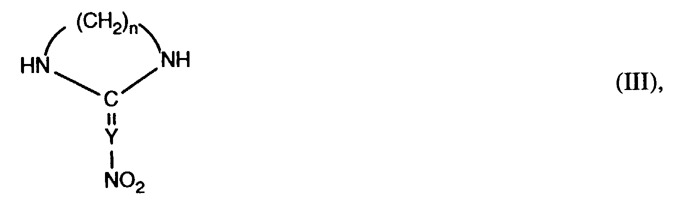 worin n und Y die unter Formel I angegebene Bedeutung haben, umsetzt.

2. Verfahren gemäss Anspruch 1 zur Herstellung einer Verbindung der Formel I, worin R₁ und R₂ unabhängig voneinander Wasserstoff oder Chlor bedeuten.

3. Verfahren gemäss Anspruch 2 zur Herstellung einer Verbindung der Formel I, worin R₁ Chlor und R₂ Wasserstoff oder Chlor bedeutet.

4. Verfahren gemäss Anspruch 1 zur Herstellung einer Verbindung der Formel I, worin R₃ Wasserstoff bedeutet.

5. Verfahren gemäss Anspruch 1 zur Herstellung einer Verbindung der Formel I, worin R₁ Chlor und R₂ und R₃ Wasserstoff bedeuten.

6. Verfahren gemäss Anspruch 1 zur Herstellung einer Verbindung ausgewählt aus der Reihe:
3-(2-Nitromethyliden-imidazolidin-1-ylmethyl)-pyridin-N-oxid,
2-Chlor-5-(2-nitromethyliden-imidazolidin-1-ylmethyl)-pyridin-N-oxid und
2-Chlor-5-(2-nitroimino-imidazolidin-1-ylmethyl)-pyridin-N-oxid.

7. Schädlingsbekämpfungsmittel, welches als aktive Komponente mindestens ein 3-Picolin-N-oxid der Formel I gemäss Anspruch 1 enthält.

8. Mittel gemäss Anspruch 7, dadurch gekennzeichnet, dass es neben dem Wirkstoff der Formel I noch mindestens einen Trägerstoff enthält.

9. Verfahren zur Bekämpfung von pflanzenschädigenden Insekten, dadurch gekennzeichnet, dass man die Schädlinge oder ihren Lebensraum mit einer wirksamen Menge einer Verbindung gemäss Anspruch 1 der Formel I behandelt.

10. Eine Verbindung gemäss Anspruch 1 der Formel I zur Anwendung in einem Verfahren zur Bekämpfung von Schädlingen an Tieren und Pflanzen.

11. Verbindung zur Anwendung gemäss Anspruch 10, dadurch gekennzeichnet, dass es sich bei den Schädlingen um pflanzenschädigende Insekten handelt.

## Claims (Claims for the following Contracting State(s): BE, CH, DE, DK, FR, GB, GR, IT, LI, LU, NL)

1. A 3-picoline-N-oxide of the formula 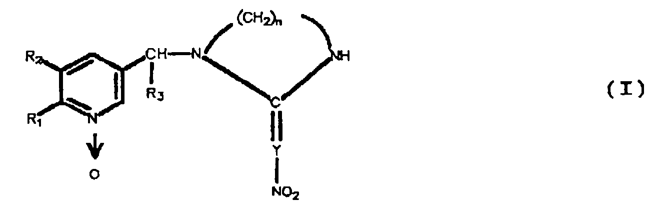 wherein
R₁ and R₂ independently of each other are hydrogen or halogen,
R₃ is hydrogen or C₁-C₄alkyl,
n is the number two or three, and
Y is nitrogen or the methine group.

2. A compound according to claim 1 of formula I wherein R₁ and R₂ independently of each other are hydrogen or chlorine.

3. A compound according to claim 2 of formula I wherein R₁ is chlorine and R₂ is hydrogen or chlorine.

4. A compound according to claim 1 of formula I wherein R₃ is hydrogen.

5. A compound according to claim 1 of formula I wherein R₁ is chlorine and R₂ and R₃ are hydrogen.

6. A compound according to claim 1 selected from the group:
3-(2-nitromethylidene-imidazolidin-1-ylmethyl)-pyridine-N-oxide,
2-chloro-5-(2-nitromethylidene-imidazolidin-1-ylmethyl)-pyridine-N-oxide and
2-chloro-5-(2-nitroimino-imidazolidin-1-ylmethyl)-pyridine-N-oxide.

7. A process for the preparation of a compound of formula I according to claim 1, which comprises reacting a halo-3-picoline-N-oxide of the formula 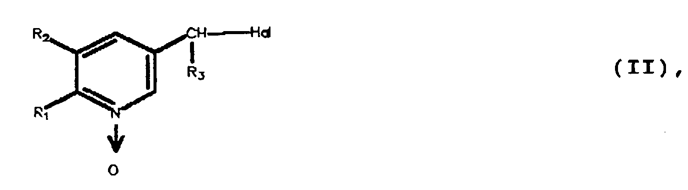 wherein R₁, R₂ and R₃ are as defined under formula I and Hal is halogen, in the presence of an acid-binding agent with a cyclic amine of the formula 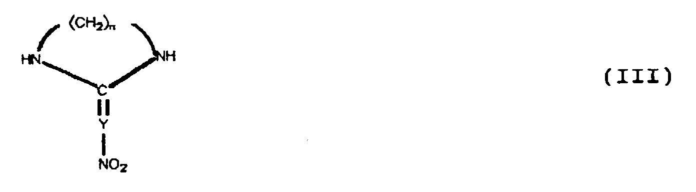 wherein n and Y are as defined under formula I.

8. A pesticidal composition comprising as active ingredient at least one 3-picoline-N-oxide of formula I according to claim 1.

9. A composition according to claim 8 comprising, in addition to the compound of formula I, at least one carrier.

10. A method of controlling plant-destructive insects, which comprises treating the pests or their locus with an effective amount of a compound according to claim 1 of formula I.

11. A compound according to claim 1 of formula I for use in a method of controlling pests on animals and plants.

12. A compound for use according to claim 11, wherein the pests are plant-destructive insects.

## Claims (Claims for the following Contracting State(s): ES)

1. A process for the preparation of a 3-picoline-N-oxide of the formula 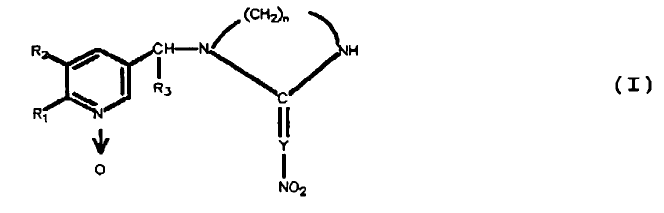 wherein
R₁ and R₂ independently of each other are hydrogen or halogen,
R₃ is hydrogen or C₁-C₄alkyl,
n is the number two or three, and
Y is nitrogen or the methine group,
which comprises reacting a halo-3-picoline-N-oxide of the formula 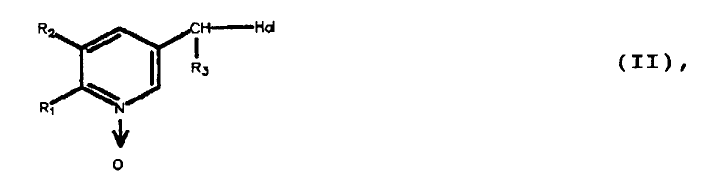 wherein R₁, R₂ and R₃ are as defined under formula I and Hal is halogen, in the presence of an acid-binding agent with a cyclic amine of the formula 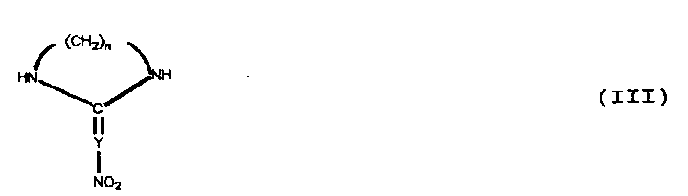 wherein n and Y are as defined under formula I.

2. A process according to claim 1 for the preparation of a compound of formula I wherein R₁ and R₂ independently of each other are hydrogen or chlorine.

3. A process according to claim 2 for the preparation of a compound of formula I wherein R₁ is chlorine and R₂ is hydrogen or chlorine.

4. A process according to claim 1 for the preparation of a compound of formula I wherein R₃ is hydrogen.

5. A process according to claim 1 for the preparation of a compound of formula I wherein R₁ is chlorine and R₂ and R₃ are hydrogen.

6. A process according to claim 1 for the preparation of a compound selected from the group:
3-(2-nitromethylidene-imidazolidin-1-ylmethyl)-pyridine-N-oxide,
2-chloro-5-(2-nitromethylidene-imidazolidin-1-ylmethyl)-pyridine-N-oxide and
2-chloro-5-(2-nitroimino-imidazolidin-1-ylmethyl)-pyridine-N-oxide.

7. A pesticidal composition comprising as active ingredient at least one 3-picoline-N-oxide of formula I according to claim 1.

8. A composition according to claim 7 comprising, in addition to the compound of formula I, at least one carrier.

9. A method of controlling plant-destructive insects, which comprises treating the pests or their locus with an effective amount of a compound according to claim 1 of formula I.

10. A compound according to claim 1 of formula I for use in a method of controlling pests on animals and plants.

11. A compound for use according to claim 10, wherein the pests are plant-destructive insects.

## Revendications (Revendications pour l'(les) Etat(s) contractant(s) suivant(s): BE, CH, DE, DK, FR, GB, GR, IT, LI, LU, NL)

1. Un N-oxyde de 3-picoline de formule 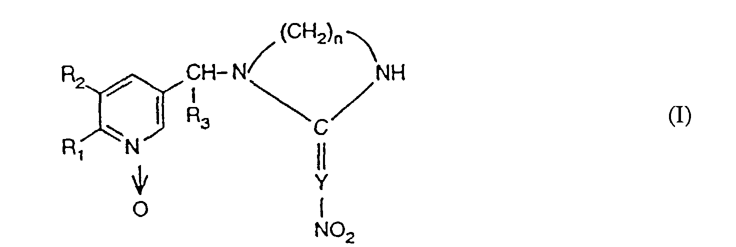 dans laquelle
R₁ et R₂ représentent chacun, indépendamment l'un de l'autre, l'hydrogène ou un halogène,
R₃ représente l'hydrogène ou un groupe alkyle en C₁-C₄,
n est égal à 2 ou 3 et
Y représente l'azote ou le groupe méthine.

2. Un composé selon la revendication 1, répondant à la formule I dans laquelle R₁ et R₂ représentent chacun, indépendamment l'un de l'autre, l'hydrogène ou le chlore.

3. Un composé selon la revendication 2, répondant à la formule I dans laquelle R₁ représente le chlore et R₂ l'hydrogène ou le chlore.

4. Un composé selon la revendication 1, répondant à la formule I dans laquelle R₃ représente l'hydrogène.

5. Un composé selon la revendication 1, répondant à la formule I dans laquelle R₁ représente le chlore et R₂ et R₃ l'hydrogène.

6. Un composé selon revendication 1, choisi parmi les suivants :
N-oxyde de la 3-(2-nitrométhylidène-imidazolidine-1-ylméthyl)-pyridine,
N-oxyde de la 2-chloro-5-(2-nitrométhylidène-imidazolidine-1-ylméthyl)-pyidine, et
N-oxyde de la 2-chloro-5-(2-nitroimino-imidazolidine-1-ylméthyl)-pyridine.

7. Procédé de préparation d'un composé de formule I selon la revendication 1, caractérisé en ce que l'on fait réagir un N-oxyde d'halogéno-3-picoline de formule 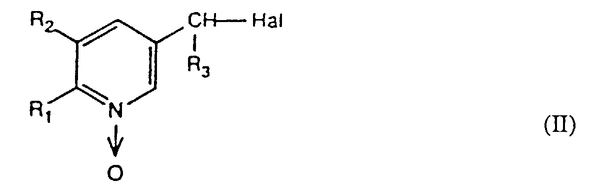 dans laquelle R₁, R₂ et R₃ ont les significations indiquées en référence à la formule I et Hal représente un halogène, en présence d'un capteur d'acides, avec une amine cyclique de formule 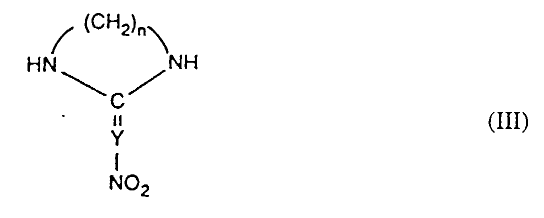 dans laquelle n et Y ont les significations indiquées en référence à la formule I.

8. Produit parasiticide contenant au moins un composant actif qui consiste en un N-oxyde de 3-picoline de formule I selon la revendication 1.

9. Produit selon revendication 8, caractérisé en ce que, en plus de la substance active de formule I, il contient encore au moins un véhicule.

10. Procédé pour combattre les insectes nuisibles pour les végétaux, caractérisé en ce que l'on traite les parasites ou leur habitat par une quantité efficace d'un composé de formule I selon revendication 1.

11. Un composé selon revendication 1, répondant à la formule I, pour l'utilisation dans un procédé pour combattre les parasites des animaux et des végétaux.

12. Un composé pour l'utilisation selon revendication 11, caractérisé en ce que les parasites sont des insectes nuisibles pour les végétaux.

## Revendications (Revendications pour l'(les) Etat(s) contractant(s) suivant(s): ES)

1. Procédé de préparation d'un N-oxyde de 3-picoline de formule 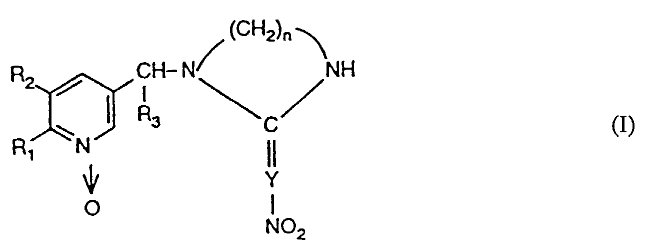 dans laquelle
R₁ et R₂ représentent chacun, indépendamment l'un de l'autre, l'hydrogène ou un halogène,
R₃ représente l'hydrogène ou un groupe alkyle en C₁-C₄,
n est égal à 2 ou 3, et
Y représente l'azote ou le groupe méthine, caractérisé en ce que l'on fait réagir un N-oxyde d'halogéno-3-picoline de formule 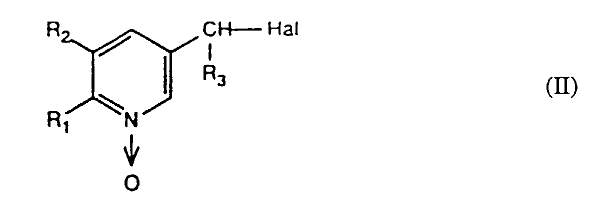
dans laquelle R₁, R₂ et R₃ ont les significations indiquées en référence à la formule I et Hal représente un halogène, en présence d'un capteur d'acides, avec une amine cyclique de formule 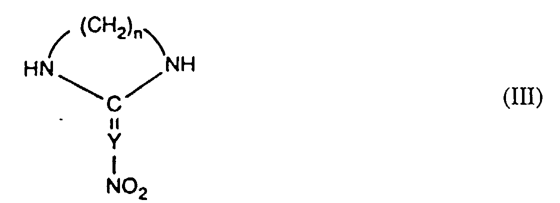 dans laquelle n et Y ont les significations indiquées en référence à la formule I.

2. Procédé selon la revendication 1 pour préparer un composé de formule I dans laquelle R₁ et R₂ représentent chacun, indépendamment l'un de l'autre, l'hydrogène ou le chlore.

3. Procédé selon la revendication 2, pour préparer un composé de formule I dans laquelle R₁ représente le chlore et R₂ l'hydrogène ou le chlore.

4. Procédé selon la revendication 1, pour préparer un composé de formule I dans laquelle R₃ représente l'hydrogène.

5. Procédé selon la revendication 1, pour préparer un composé de formule I dans laquelle R₁ représente le chlore et R₂ et R₃ l'hydrogène.

6. Procédé selon la revendication 1, pour préparer un composé choisi parmi les suivants :
N-oxyde de la 3-(2-nitrométhylidène-imidazolidine-1-ylméthyl)-pyridine,
N-oxyde de la 2-chloro-5-(2-nitrométhylidène-imidazolidine-1-ylméthyl)-pyridine, et
N-oxyde de la 2-chloro-5-(2-nitroimino-imidazolidine-1-ylméthyl)-pyridine.

7. Produit parasiticide contenant au moins un composant actif consistant en un N-oxyde de 3-picoline de formule I selon la revendication 1.

8. Produit selon la revendication 7, caractérisé en ce que, en plus de la substance active de formule I, il contient encore au moins un véhicule.

9. Procédé pour combattre les insectes nuisibles pour les végétaux, caractérisé en ce que l'on traite les parasites ou leur habitat par une quantité efficace d'un composé de formule I selon la revendication 1.

10. Composé selon la revendication 1 et répondant à la formule I pour l'utilisation dans un procédé pour combattre les parasites des animaux et des végétaux.

11. Un composé pour l'utilisation selon la revendication 10, caractérisé en ce que les parasites sont des insectes nuisibles pour les végétaux.
